# EUROPEAN PATENT APPLICATION

(11) **EP 1 748 050 A1**
(43) Date of publication of application: **31.01.2007**
(21) Application number: 05076747.4
(22) Date of filing: 28.07.2005
(51) Int. Cl.: C07K 14/47, C12N 15/62, A61K 38/17

(54) **Targeting-enhanced activation of galectins**

(71) Applicant: Rijksuniversiteit Groningen, 9712 CP Groningen (NL)
(72) Inventor: Bremer, Edwin, 9714 HB Groningen (NL); Helfrich, Wijnand, 9713 GZ Groningen (NL)
(74) Representative: Winckels, Johannes Hubertus F.

(57) **Abstract**

The invention relates to lectin-binding proteins and their therapeutic use, inter alia in the field of immunology and oncology. In particular, it relates to the targeting and targeting-enhanced multimerization and activation of galectins. Provided is a galectin-conjugate comprising at least one galectin molecule conjugated to a non-galectin cell targeting means. Exemplary targeting means include targeting means capable of binding to EGP2, a pancarcinoma associated cell surface target antigen, CD antigen, such as CD7 or CD38, or a TNF family member, such as TRAIL-R. The targeting means may comprise an antibody or a functional fragment thereof, preferably a single chain variable antibody fragment (scFv). Also provided is the use of a galectin-conjugate for the treatment of a disease, like cancer or an immune disorder, such as auto-immune disease, allergic disorder, autoimmune encephalomyelitis, arthritis, colitis, hepatitis, asthma, multiple sclerosis, transplant rejection, Graft-versus-host disease (GVHD) and inflammatory bowel disease.

## Description

The invention relates to lectin-binding proteins and their therapeutical use, inter alia in the field of immunology and oncology. In particular, it relates to the targeting and targeting-induced multimerization and subsequent activation of galectins.

Galectins are members of a highly conserved family of beta-galactoside-binding animal lectins. Members of this family are distinguished from other lectins by the presence of a conserved carbohydrate recognition domain which has an affinity for poly-N-linked-acetyl-lactosamine-rich glycoproteins. The majority of galectins bind to sugar molecules in a sulfhydryl-dependent manner and are often referred to as S-type lectins, however this property is not required for membership in this class.

Presently, at least 15 members have been identified and additional homologues are likely to be discovered. Given their conservation throughout animal evolution, it is not surprising that they could play key roles in innate and adaptive immune responses, through sugar-dependent and -independent mechanisms. Recently, it has become increasingly clear that galectins can differentially affect cellular activation and function. These biological effects attracted attention of researchers in cell biology, biochemistry, tumor biology, oncology, glycobiology and immunology, not only in the mode of action of galectins, but also in their role as putative modulators of immune surveillance, apoptosis, cell adhesion and chemotaxis. Intriguingly, it has been recently realized that the same galectin might exert pro-or anti-inflammatory effects depending on multiple factors, including subcellular localization and the type of target cell. For reviews, see for example Liu, "Regulatory roles of galectins in the immune response" Int. Arch. Allergy Immunol. 2005, 136(4):385-400; Liu and Rabinovich, "Galectins as modulators of tumor progression." Nat. Rev. Cancer. 2005, 5(1):29-41; Rubinstein et al., "The role of galectins in the initiation, amplification and resolution of the inflammatory response." Tissue Antigens. 2004, 64(1):1-12; and Yang and Liu, "Galectins in cell growth and apoptosis", Cell. Mol. Life Sci. 60 (2003); 267-276.

Galectins are also known in the art as galaptins, S-Type lectins,; D-Galactoside-Binding Lectins, Galactose-Binding Lectins, beta-D-Gal(1-3)D-GalNAc Specific Lectins; beta-D-Galactosyl-Specific Lectins, beta-Galactoside Binding Lectins or beta-galactoside binding proteins (βGBPs).

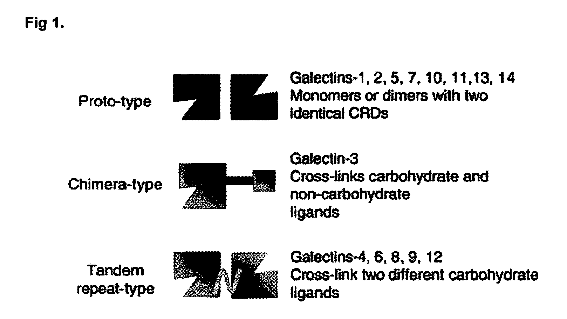

Members of the galectin family are composed of one or two carbohydrate-recognition domains (CRDs) of approximately 130 amino acids (Fig. 1). Regarding the biochemical structure, some galectins contain one CRD (proto-type) and exist as monomers (galectin-5, -7 and -10) or dimers (galectin-1, -2, -11, -13 and -14), whereas other galectins, such as galectin-4, -6, -8, -9 and -12, contain two CRDs connected by a short linker region (tandem repeat). In contrast, galectin-3 uniquely occurs as a "chimeric" protein with one CRD and an additional non-lectin domain, which is involved in the oligomerization of this protein (Fig. 1). It has been suggested that multivalency of individual members of the galectin family with respect to their CRD's and their crosslinking properties might determine different biological responses by inducing aggregation of specific cell-surface glycoreceptors, which - in many cases - are associated with different signal transduction events.

The potential of some of these galectin family members in the treatment of (auto)-immune diseases has been investigated by several research groups. Most scientific attention is focused on the first identified member of this family, Galectin-1. Galectin-1 is biologically active as a homodimer and shows a host of (immuno) regulatory functions such as cell adhesion, cell growth, neoplastic transformation, migration, T cell maturation and the induction of apoptosis in macrophages, thymocytes, and T cells.

*In vivo* administration of recombinant Galectin-1 has been shown to ameliorate disease activity in mouse models of arthritis, Graft versus Host disease, hepatitis, nephritis, inflammatory bowel disease, and multiple sclerosis (Rabinovich et al, J Exp Med. 1999 Aug 2;190(3):385-98; Baum et al, Clin Immunol. 2003 Dec;109(3):295-307; Santucci et al, Hepatology. 2000 Feb;31(2):399-406; Tsuchiyama et al, Kidney Int. 2000 Nov; 58(5):1941-52; Santucci, Gastroenterology. 2003 May;124(5):1381-94; Offner et al, J of Neuroimmunol 1990; 28:177-184). Furthermore, Galectin-3, has recently been shown to inhibit bronchial obstruction and inflammation in an experimental model of asthma by comparing the allergic airway response in galectin-3-deficient (gal3(-/-)) mice and wild-type (gal3(+/+)) mice (Zuberi et al., Am. J. Pathol. 2004;165(6):2045).

Interestingly, various galectin family members have been shown to play an important role in tumor development and anti-tumor responses. For instance, galectin-1 is expressed in a number of different tumor types and may modulate the immune response to the tumor by elimination of infiltrating T cells. Reversely, several T-ALL leukemia cell lines have been shown to be highly sensitive to apoptosis induction by Galectin-1. Very recently it was shown that multidrug resistant tumor cells of various origins are sensitive to apoptosis induction by Galectin-1 (Ravatn et al., Cancer Res. 2005;65(5):1631-4). Thus, the effect exerted by a galectin, either pro- or anti-apoptotic, appears to be cell-type dependent.

For most activities the physiologically active form of galectin-1 has been reported to be a homodimer with a subunit molecular mass of 14.5 kDa (Cho and Cummings, 1995a,b; Perillo et al., 1995), while the monomeric form is hardly biologically active. The monomeric and the dimeric form are found in a reversible equilibrium with a K_{d} of ≈ 7 µM (Cho and Cummings, 1995a,b). Based on this low affinity, the *in vivo* efficacy of galectin-1 is limited because at lower concentrations the equilibrium is rapidly shifted towards the inactive monomeric form. In the studies mentioned above, galectin subunits were used which were not covalently linked. Several mouse models showed that up to 4 mg/kg were needed to obtain a therapeutic effect, largely precluding therapeutic application in humans. Thus, high amounts of galectin are needed to reach the critical concentration of the active dimer required for biological efficacy as a therapeutic agent.

This problem has been addressed in the art for galectin-1. Bättig et al. (Mol. Immunol. 2004;41(1):9-18) describe the design of a structurally optimized form of galectin-1, wherein two monomers are genetically fused from C to N via a two-glycine (GG) linker moiety. The artificial covalent dimer was found to be efficiently secreted and was 3-10 times more potent at inducing apoptosis in an *in vitro* assay system when compared to wild-type galectin-1.

It is an object of the present invention to provide alternative methods to enhance the *in vivo* efficacy of galectins. Furthermore, it is an object of the invention to provide a therapeutic use of galectin with a high *in vivo* efficacy without compromising target cell specificity.

This goal is met by the provision of a galectin-conjugate comprising at least one galectin molecule conjugated to a non-galectin cell targeting means. The recruitment of galectin to a target cell concentrates its presence at the desired site of action and thus reduces the amount required to exert a biological effect.

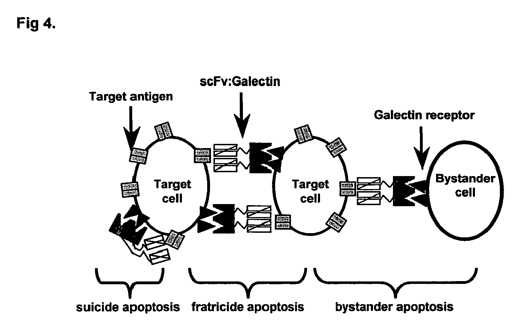

Galectin-conjugates of the invention exert their full biological (galectin-mediated) activity after specific binding to a target cell, e.g. activated T-cells or tumor cells. The novel galectin conjugates have a strongly enhanced activity compared to native, non-targeted galectins as well as to non-targeted artificial dimers. The specificity of the targeting means allows for directing the galectin activity to a pre-selected cell type. Given the strong cell-dependent effect of galectins, this is of particular relevance for the therapeutic application of galectins. For instance, when targeting a galectin to induce apoptosis in a desired cell type, e.g. a tumor cell, unwanted side effects on other cell types can be reduced by choosing one or more targeting means which do(es) bind to the tumor cells yet not to cells which can be negatively or adversely affected by the galectin. Therapeutic galectin conjugates of the invention can be specifically designed and optimized for a given disease. It was found that conjugation of a targeting means to galectin dramatically enhances the biological efficacy of galectin, conceivably by cell surface accretion. Figure 4 and the legend thereto further illustrate the proposed mechanism(s) of action of a galectin-conjugate as disclosed herein.

The conjugation to a targeting means is of benefit for galectins which are active as multimers (e.g. galectin-1) as well as for other galectin subtypes, e.g. galectin-3. With the provision of the novel galectin conjugates, the invention thus provides for target cell-restricted therapeutic application of galectins in the treatment of various diseases.

The term "cell targeting means" as used herein refers to a moiety or substance capable of directing the galectin molecule to a target cell by virtue of its ability to bind or interact with a molecule expressed on the surface of the target cell. The cell targeting means can be of any nature. It is for instance a synthetic compound capable of binding to a target cell, which compound may be identified by high-throughput screening using a library of chemical compounds. It can be a mimetic of a naturally occurring molecule which can bind to or interact with a molecule expressed on the surface of the target cell. The targeting means can be conjugated to either the N- or C-terminus of the galectin molecule, either directly or via a linker sequence, optionally via a dimerization domain (see further below).

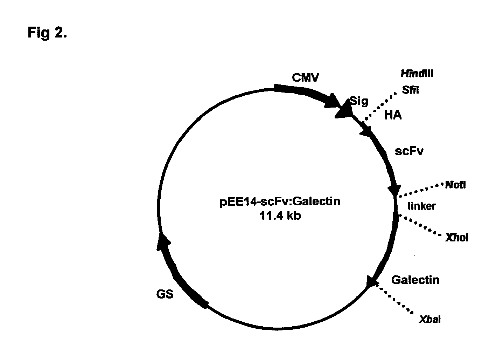

In one embodiment, the targeting means is a proteinaceous substance. The targeting means can be conjugated to the galectin molecule in any suitable fashion, e.g. by chemical coupling or by genetic fusion. In a preferred embodiment, the invention provides a galectin-conjugate comprising a proteinaceous targeting means genetically fused to a galectin molecule. Such a galectin-conjugate can be readily produced by the recombinant expression of an expression plasmid comprising a nucleic acid construct encoding a galectin-conjugate fusion protein. Also provided herein is a nucleic acid construct encoding a galectin-conjugate fusion protein (see also Figure 2). The fusion of a galectin molecule to a cell targeting means may be direct or via a linker sequence. The use of a linker sequence can be advantageous to achieve an optimal spatial organization of both the galectin molecule and the cell targeting means. The linker sequence can be small, e.g. from 1 to about 15 amino acids, medium (about 15 to 50 amino acids) or large (50 amino acids or more, such as 75 or even 100 amino acids). Examples of useful linker sequences include selected amino acids of the IgG heavy chain constant region CH1, selected amino acids of the IgG hinge region, artificial linker peptides containing one or more of the amino acid motif GGGGS (G4S) (Huston et al. 1988, Proc Natl Acad Sci U S A.; 85(16):5879-83.) or derivatives thereof, such as GGGSS.

Furthermore, a fusion protein may comprise an affinity tag, such as HA, poly-His (6 x His), GST or c-Myc, to aid in the isolation and purification of the fusion protein upon recombinant expression. Many tags have their own characteristics. For example, poly-His-fusion proteins can bind to Nickel-Sepharose or Nickel-HRP. GST-fusion proteins can bind to glutathione-Sepharose. Therefore, a high degree of purification of fusion protein can be achieved in just one affinity purification step.

In a preferred embodiment, the cell targeting means comprises an antibody or a functional fragment thereof, preferably a single chain variable fragment (scFv). Recombinant antibodies have an increasing number of applications in biotechnology and medicine. The molecular techniques allow to generate a recombinant antibody which maintains the antigen-binding competence of the parental monoclonal antibody. The antigen binding ability of the antibody is usually conserved when only the V_{H} and V_{L} domains are connected with a polypeptide linker to constitute a single chain variable fragment (scFv) recombinant antibody. The scFv recombinant antibody contains the complete antigen-binding site of an antibody. The smaller format of the scFv (27kDa) allows significant advantages of formatting (fusion with others molecules) and manufacturing in a recombinant host cell, for example using a mammalian, yeast or bacterial production system.

Antibody fragments and peptides specific for essentially any antigen, be it a peptide, sugar, lipid, nucleic acid or whole organism etc., can be selected by methods known in the art. Peptide libraries containing large amounts of randomly synthesized peptides which can be used in selecting a suitable binding partner for an antigen of interest are commercially available. For instance, New England Biolabs offers pre-made random peptide libraries, as well as the cloning vector M13KE for construction of custom libraries. The pre-made libraries consist of linear heptapeptide and dodecapeptide libraries, as well as a disulfide-constrained heptapeptide library. The randomized segment of the disulfide-constrained heptapeptide is flanked by a pair of cysteine residues, which are oxidized during phage assembly to a disulfide linkage, resulting in the displayed peptides being presented to the target as loops. All of the libraries have complexities in excess of 2 billion independent clones. The randomized peptide sequences in all three libraries are expressed at the N-terminus of the minor coat protein pIII, resulting in a valency of 5 copies of the displayed peptide per virion. All of the libraries contain a short linker sequence (Gly-Gly-Gly-Ser) between the displayed peptide and pIII.

Of particular interest is the use of phage display technology. Many reviews on phage display are available, see for example Smith and Petrenko [1997] Chem. Rev. 97:391-410. Briefly, phage display technology is a selection technique in which a library of variants of a peptide or human single-chain Fv antibody is expressed on the outside of a phage virion, while the genetic material encoding each variant resides on the inside. This creates a physical linkage between each variant protein sequence and the DNA encoding it, which allows rapid partitioning based on binding affinity to a given target molecule (antibodies, enzymes, cell-surface receptors, etc.) by an *in vitro* selection process called panning. In its simplest form, panning is carried out by incubating a library of phage-displayed peptides with a plate (or bead) coated with the target (i.e. antigen of interest), washing away the unbound phage, and eluting the specifically bound phage. The eluted phage is then amplified and taken through additional binding/amplification cycles to enrich the pool in favour of binding sequences. After 3-4 rounds, individual clones are typically characterized by DNA sequencing and ELISA. The DNA contained within the desired phage encoding the particular peptide sequence can then be used as nucleic acid encoding a non-galectin cell targeting means in a nucleic acid construct encoding a galectin-conjugate of the invention. Alternatively, high-affinity binders can be selected from combinatorial libraries of designed ankyrin repeat proteins (Binz et al. Nat. Biotechnol. 2004; 22(5):575-82.

Also suitable for use as cell targeting means are iMabs (industrial Molecular affinity bodies) developed by CatchMabs BV, Wageningen, The Netherlands. IMabs are small designer proteins that can bind target molecules with both high affinity and high specificity, even under the harsh chemical conditions of the processing industry. Due to their small size, iMabs can be produced cheaply, e.g. as a fusion with a galectin molecule, in standard production micro-organisms such as *E.coli* or yeasts. A general overview of technology related to the design of specific binding proteins can be found in Binz and Pluckthun (Curr Opin Biotechnol. 2005 Jul 5; [Epub ahead of print]).

As said, a galectin-conjugate can be directed to a target cell of interest by virtue of a cell targeting means capable of binding to a target cell surface molecule. The targeting means is for instance reactive with a target cell surface molecule selected from the group consisting of cluster of differentiation (CD) antigens, ABC transporter proteins, adhesion molecules, ligands that are expressed exclusively or predominantly on one or more types of leucocytes, one or more sub-populations of T cells or B cells, cytokine receptors, growth factor receptors, molecules or complex comprising a transmembrane protein, tyrosine kinase type receptors, death receptors, serine kinase type receptors, heterotrimeric G-protein coupled receptors, receptors bound to tyrosine kinase, TNF family receptors, notch family receptors, guanylate cyclase types, tyrosine phosphatase types, decoy receptors, inhibitory receptors and adhesion receptors or any cell surface receptor-ligand cooperating with such a receptor.

In a preferred embodiment, a galectin-conjugate comprises a targeting means capable of binding to a CD antigen, such as CD7 or CD38, or a TNF family member, such as TRAIL-R. For instance, provided herein is a galectin conjugated to a scFv reactive with CD7 or CD38.

In one aspect, the invention relates to a galectin-conjugate comprising a cell targeting means capable of interacting with a cell surface receptor on a target cell, which receptor can undergo internalization. This allows for co-internalization of the galectin-conjugate together with the receptor, such that the galectin molecule reaches the intracellular environment where it can exert its action. It has been shown that the same galectin (e.g. galectin-3) may exert different and contrasting effects whether they act extracellularly or intracellularly (Yang et al., Proc Natl Acad Sci U S A. 1996; 25;93(13):6737-42; Fukumori et al., Cancer Res. 2003 Dec 1;63(23):8302-11). Conjugation to a cell targeting means which allows for uptake of the galectin molecule enhances the intracellular effect. This can be used to enhance the intracellular effect of a galectin molecule relative to the extracellular effect. Many receptors are known in the art which undergo internalization. Exemplary receptors include the transferrin receptor (TfR or CD71), the EGF receptor (EGF-R), the stem cell factor (SCF) receptor KIT, the mannose-6-phosphate receptor, and the like.

In one aspect of the invention, the targeting means allows for cancer-cell selective targeting of a galectin. This can be achieved by choosing a targeting means which is reactive with a surface molecule that is overexpressed on tumor cells as compared to non-tumor cells. Suitable targeting antigens to achieve cancer-cell selective targeting of galectin include the Epidermal growth factor receptor 3 (EGF-R3) and EGP-2.

A galectin molecule can also be provided with a targeting means that is capable of binding simultaneously to a multiplicity of, either identical or distinct, cell surface molecules. The use of a bi- or multi-functional targeting means allows to increase the binding affinity and/or specificity to a target cell. For example, a galectin-conjugate comprises a galectin provided with a first scFv capable of binding molecule A, flanked, optionally spaced by a linker sequence, by a second scFv capable of binding to molecule B. If molecules A and B are present in close proximity of each other on the same target cell, said bifunctional galectin-conjugate can bind via both targeting means to the target cell. This can enhance the binding avidity and /or specificity of a galectin-conjugate to a target cell.

The term 'galectin molecule" as used herein refers to any known and yet to be discovered member of the galectin family of secreted galectins containing a conserved carbohydrate recognition domain (CRD). Included are galectin-1,-2,-3,-4,-5,-6,-7,-8,-9,-10,-11,-12,-13,-14 and -15. The nucleotide and amino acid sequences for human and/or other species of these known galectins are available from public databases.

In a preferred embodiment, the invention provides a galectin-conjugate comprising a galectin molecule which are active as multimers. Examples are galectin-1, -2, -5, -7, -10, -11, -13 and -14. Targeting of these galectins to target cells has the additional advantage that cell surface accretion enhances multimerization-induced activation, thereby even further enhancing the *in vivo* efficacy. Thus, galectin activity towards a target cell is still controlled by the extent of lectin-induced dimerization at the target cell surface. This is in contrast to artificial dimers, e.g. the galectin-1 dimer described by Bättig et al., which is always active, irrespective of its local environment or cellular context. As a consequence, it is conceivable that *in vivo* administration of constitutively active galectin dimers which are not specifically targeted to the desired target cells cause unwanted side effects, in particular on cells that are normally not 'encountered' by galectins.

The invention provides not only conjugates of naturally occurring galectins, but also of non-naturally occurring galectin molecules, also referred to as galectin mutants or modified galectins. Galectin mutants have at least one amino acid substitution, deletion or addition when compared to the naturally occurring amino acid sequence. Galectin mutants having increased stability (e.g. less susceptible to oxidation) and/or increased activity compared to wild-type galectin are also encompassed. Galectin mutants can be active or inactive. A galectin deletion mutant is for instance a truncated galectin. A truncated galectin can act as a therapeutic agent by acting as a competitive inhibitor of naturally present galectins. For example, it is thought that the chimera type galectin-3 plays a role in cancer formation by promoting cell-to-cell adhesion. Removal of the domain of galectin-3 that normally allows cells to stick to each other yields a modified galectin which occupies the site on a cell's surface, thereby blocking normal, endogenous galectin-3 from binding. This stops cells from adhering to each other. It was shown that the modified protein more than halved the number of mice that developed metastatic tumors. Cancer implanted into the mice spread to the lymph nodes or other organs in 11 of the 20 control mice given sham injections, but only four of the 20 mice given the truncated protein (John et al., "Truncated galectin-3 inhibits tumor growth and metastasis in orthotopic nude mouse model of human breast cancer." Clin Cancer Res. 2003 Jun;9(6):2374-83).

A galectin-conjugate may comprise a dimer of galectin molecules, at least one of which is conjugated to a cell targeting means. The galectin molecules of said dimer can be covalently or non-covalently linked. A noncovalent linkage between galectin molecules can be achieved by means of a dimerization domain, such as a leucine zipper domain. The leucine zipper is composed of two α-helices. Each α-helix has a basic region at the N-terminal end that contains several positively charged residues that, when present in transcription factors, can interact with the major groove of the DNA. Toward the C-terminal of the α-helices, the dimerization region is found. A covalent linkage can be achieved through a GG linker as described in the art by Bättig et al. for galectin-1.

In another embodiment, use is made of an S-S zipper (De Kruif J, Logtenberg T. J Biol Chem. 1996 Mar 29;271(13):7630-4. "Leucine zipper dimerized bivalent and bispecific scFv antibodies from a semi-synthetic antibody phage display library". However, SS-zippers require rounds of oxidation and reduction, which may be disadvantageous for the sulfhydryl-dependent lectin binding activity of galectin. Therefore, zippers which do not rely on S-S/S-H functionality are preferred.

In another embodiment, use is made of the so-called knobs into holes technology: 'Knobs-into-holes' was originally proposed by Crick in 1952 as a model for the packing of amino acid side chains between adjacent alpha-helices. 'Knobs-into-holes' has been demonstrated in the art to be an effective design strategy for engineering antibody heavy chain homodimers for heterodimerization. In a study by Ridgeway et al. (Protein Eng. 1996 Jul;9(7):617-21) directed at heavy chain heterodimerization, a 'knob' variant was first obtained by replacement of a small amino acid with a larger one in the CH3 domain of a CD4-IgG immunoadhesin: T366Y. The knob was designed to insert into a 'hole' in the CH3 domain of a humanized anti-CD3 antibody created by judicious replacement of a large residue with a smaller one. Alternatively, use can be made of Fab antibody fragments. In that case, two chains are required, of which one has the modular structure: VL-Ckappa-Gal1 and the other has the modular structure VH-CH1-Gal1. The Ckappa and the CH1 modules can interact with each other via a S=S bridge.

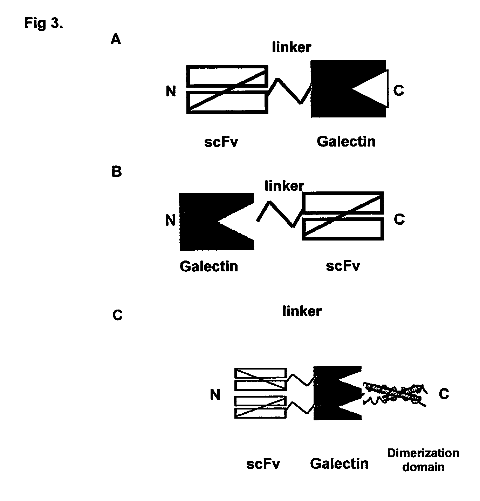

A dimerization domain can be attached to the N-terminal or C-terminal end of a galectin molecule. Similar to what is described above for the conjugation site of the cell targeting means, it may be attached directly to the galectin molecule or via a linker moiety. In one embodiment, a galectin molecule is conjugated via a linker at its N-terminus to a cell targeting means, like a scFv, and via its C-terminus to a dimerization domain, e.g. e leucine zipper. This galectin-conjugate can efficiently dimerize with another conjugate of similar configuration (see Fig. 3C). However, other configurations can also be suitably used, even wherein both the targeting means and the dimerization domain are conjugated to either the N- or C-terminal of the galectin molecule (see also the legend to Figure 3).

Preferably, both galectin monomers within a dimer are conjugated to a cell targeting means, as this further enhances the beneficial effect of targeting. In one embodiment, a galectin-conjugate comprises a dimer of galectins wherein each galectin is conjugated to an identical cell targeting means. In another embodiment, each galectin molecule within a galectin-conjugate according to the invention is conjugated to a distinct cell targeting means. This allows for an increased targeting specificity, for example to a subset of (tumor) cells which are characterized by the (over)expression of two distinct cell surface molecules.

Furthermore, the invention relates to a method for preparing a galectin-conjugate of the invention. In one embodiment, the galectin molecule(s) is/are produced according to standard procedures for the recombinant production in a host cell, e.g. a bacterial or a mammalian host cell transfected with a nucleic acid construct encoding the galectin of interest. Following purification, the galectin can be conjugated to a desired cell targeting means, which can be a proteinaceous or a non-proteinaceous substance. Methods for conjugation are well known in the art, like chemical coupling using a coupling agent such as succimide.. Preferably, the coupling reaction does not involve an oxidation step since galectins generally require a reducing environment for its carbohydrate binding function, which function is destroyed in oxidizing conditions. In case the cell targeting means is a proteinaceous substance, it is preferred to prepare the galectin-conjugate as a fusion protein. To that end, a fusion gene construct is prepared comprising a stretch of nucleic acids encoding a galectin cloned in frame with a stretch of nucleic acid encoding a cell-targeting means, such as a scFv antibody fragment. The cell targeting means, e.g. a scFv, may be conjugated either to the N-terminus or to the C-terminus of the galectin molecule (see Figure 3 and legend thereof).

In a further aspect, the invention provides a pharmaceutical composition comprising a galectin-conjugate according to the invention and a pharmaceutically acceptable carrier. A pharmaceutical composition comprises an active substance a galectin-conjugate, in admixture with pharmaceutically acceptable auxiliaries and optionally other therapeutic agents. The auxiliaries must be "acceptable" in the sense of being compatible with the other ingredients of the composition and not deleterious to the recipients thereof. Other therapeutic agents of interest are for example anti-inflammatory or anti-cancer agents known in the art.

Compositions include e. g. those suitable for oral, sublingual, subcutaneous, intravenous, intramuscular, local, or rectal administration, and the like, all in unit dosage forms for administration. Of particular interest are sustained release formulations. For oral administration, the active ingredient may be presented as discrete units, such as tablets, capsules, powders, granulates, solutions, suspensions, and the like. For parenteral administration, the pharmaceutical composition of the invention may be presented in unit-dose or multi-dose containers, e. g. injection liquids in predetermined amounts, for example in sealed vials and ampoules, and may also be stored in a freeze dried (lyophilized) condition requiring only the addition of sterile liquid carrier, e. g. water, prior to use.

Mixed with such pharmaceutically acceptable auxiliaries, e. g. as described in the standard reference, Gennaro, A. R. et al., Remington: The Science and Practice of Pharmacy (20th Edition., Lippincott Williams &Wilkins, 2000, see especially Part 5 : Pharmaceutical Manufacturing), the galectin-conjugate may be compressed into solid dosage units, such as pills, tablets, or be processed into capsules or suppositories. By means of pharmaceutically acceptable liquids the active substance can be applied as a fluid composition, e. g. as an injection preparation, in the form of a solution, suspension, emulsion, or as a spray, e. g. a nasal spray.

For making solid dosage units, the use of conventional additives such as fillers, colorants, polymeric binders and the like is contemplated. In general any pharmaceutically acceptable additive which does not interfere with the function of the galectin-conjugate can be used. Suitable carriers with which the active agent of the invention can be administered as solid compositions include lactose, starch, cellulose derivatives and the like, or mixtures thereof, used in suitable amounts. For parenteral administration, aqueous suspensions, isotonic saline solutions and sterile injectable solutions may be used, containing pharmaceutically acceptable dispersing agents and/or wetting agents, such as propylene glycol or butylene glycol.

Also provided is the use of a galectin-conjugate as disclosed herein for the treatment of a disease. Galectin-conjugates are suitably used for the treatment or profylaxis of a condition that would benefit from galectin therapy, comprising administering to a subject (animal/human) in need thereof a therapeutically effective dose of a pharmaceutical composition described herein above. In particular, the use is provided of a galectin-conjugate according to the invention as an apoptosis-inducing or immunosuppressive (anti-inflammatory) agent. In one embodiment, the condition to be treated (either therapeutically or prophylactically) with a galectin-conjugate comprises the treatment of an (auto)immune disorder. Exemplary immune disorders that could benefit from galectin treatment include an acute or chronic inflammatory disease, such as auto-immune disease, allergic disorder, autoimmune encephalomyelitis, arthritis, colitis, hepatitis, asthma, multiple sclerosis, transplant rejection, Graft-versus-host disease (GVHD) and inflammatory bowel disease. The clinical potential of a galectin-conjugate is illustrated in the art, e.g. Table 1 of Rubinstein et al. (2004, Tissue Antigens 64: 1-12) provides an overview of the therapeutic effects of galectins in different animal models of experimental acute and chronic inflammation, including models for multiple sclerosis, rheumatoid arthritis and Crohn's disease. Of particular interest for use in anti-inflammatory therapy are galectin-conjugates comprising galectin-1, galectin-2 or galectin-3.

In another aspect, the invention provides a method for the treatment of cancer, comprising administering to a subject (animal/human) in need thereof a therapeutically effective dose of a pharmaceutical composition comprising a galectin-conjugate as disclosed herein. Also provided is the use of a galectin-conjugate for the manufacture of a medicament for the treatment of cancer. Various galectin family members have been shown to play an important role in tumor development and anti-tumor responses. For example, it has been shown that intracellular galectin-3 exhibits the activity to suppress drug-induced apoptosis. Resistance to apoptosis is essential for cancer cell survival and plays a role in tumor progression. Furthermore, it was shown that galectin-3 secreted by tumor cells induces apoptosis of cancer-infiltrating T-cells, thereby playing a role in the immune escape mechanism of the tumor. The use of a truncated galectin which can be targeted to a cancer cell as a galectin-conjugate, e.g. a truncated galectin-3 capable of binding to T cells but non capable of inducing apoptosis, allows to compete with the high levels of galectins secreted by tumor cells. Thereby, the galectin-conjugate can interfere with the immune escape mechanism of tumor cells.

On the other hand, several T-ALL leukemia cell lines are known to be highly sensitive to apoptosis induction by galectin-1. A galectin-1-conjugate with a targeting means to target T-ALL leukemic cell, such as anti-CD7, CD3, CD38 and others, can thus be used in the treatment of leukemia.

Also, it is reported that multidrug resistant tumor cells of various origin are sensitive to apoptosis induction by galectin-1 (Ravatn et al., "Circumventing multidrug resistance in cancer by beta-galactoside binding protein, an antiproliferative cytokine." Cancer Res. 2005 ;65(5):1631-4.) The mechanisms of drug resistance in the cancer cells that were examined include overexpression of P-glycoprotein, increased efficiency of DNA repair, and altered expression and mutation in the topoisomerase I and II enzymes. It was found that galectin-1 exerted its effect by arresting the cells in S phase prior to the activation of programmed cell death. The uniquely similar profile of response to galectin-1 by these drug-resistant cells and their parental cells extends the therapeutic potential of galectins in the treatment of cancers. Thus, a galectin-1-conjugate according to the invention can be used as alternative or adjuvant therapy in patients whose tumors are refractory to the currently available cadre of chemotherapeutic agents.

### LEGENDS TO THE FIGURES

**Fig. 1. Schematic presentation of the currently known Galectins.** (adapted from Rubinstein et al, Tissue Antigens, Vol 64, July 2004).
**Fig 2. Diagram of eukaryotic expression plasmid pEE14-scFv:Galectin.** The structural elements include **CMV**, strong CMV promoter; Sig, murine kappa light-chain leader sequence; **HA,** N-terminal HA-affinity tag, two MCS separated by an interchain linker sequence and GS, glutamine synthetase resistance gene for amplified expression in stable transfectants. The first MCS (MCS #1) contains unique *Sfi*I and *Not*I sites for rapid directional insertion of scFvs (e.g. selected by established phage-display technology). The second MCS (MCS #2) contains *Xho*1 and *Xba*I sites for the directional insertion of various Galectin family members after appropriate PCR manipulation.
**Fig 3. Schematic representation of some exemplary configurations of scFv:Galectin fusion proteins. A:** A non-galectin cell targeting means consisting of a recombinant antibody fragment (scFv) of pre-determined specificity is genetically fused to the N-terminus of a member of the human Galectin family via a flexible amino acid linker sequence. **B**: Alternatively, the scFv antibody fragment is conjugated at the C-terminal end of a Galectin family. **C**: A dimerization domain, such as a leucine zipper, is genetically fused to the N-terminal scFv-domain to force dimerization with a second galectin-conjugate. Alternatively, dimerization domains can be fused to the C-terminal region or can be inserted into the linker region. Note that the diagrams are not drawn to scale.
**Fig 4. Hypothetical principle of targeting-induced activation of scFv:Galectin.**
   Exposure of a target cell to the galectin-conjugate scFv:Galectin (or Galectin:scFv), results in the strong and selective binding of the conjugate at the cell surface of target antigen-positive cells. Subsequently, cell surface accretion results in the local activation of scFv:Galectin, which in turn results in the acquired ability to induce apoptosis in the same cell (suicide) or in a neighboring target antigen-positive cells (fratricide apoptosis) by reciprocal cross-linking of respective galectin receptors. Furthermore, cell surface-immobilized scFv:Galectin on a target cell can be exploited to induce apoptosis in neighboring tumor cells that are devoid of target antigen expression. This phenomenon is known as the bystander effect which can be used to treat diseased cells that would otherwise escape from therapy (diagram is not drawn to scale).

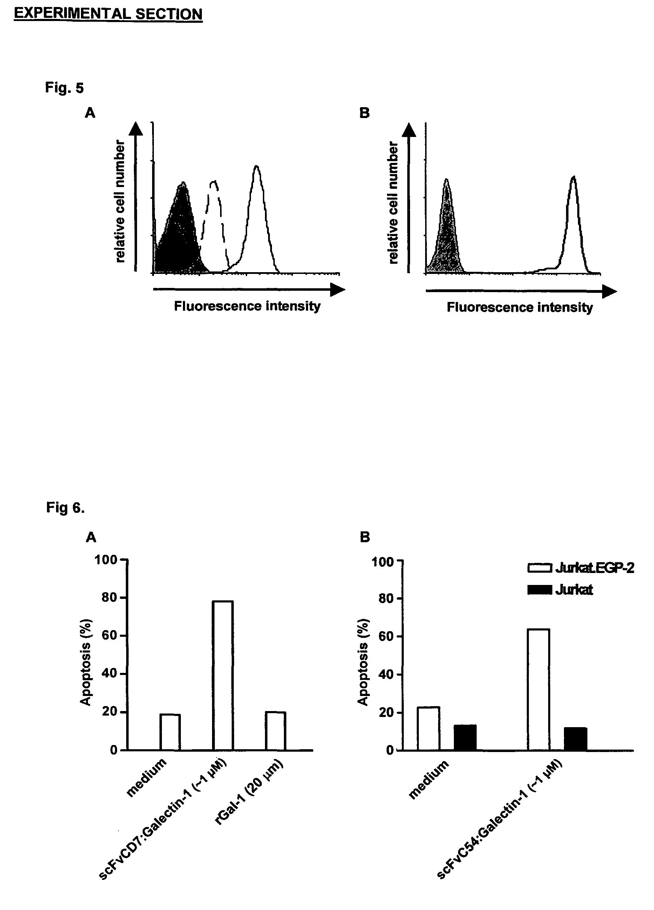

**Fig 5. Target antigen-restricted binding of tumor-targeted scFv:Galectin-1 conjugates. A:** Proof of principle for target antigen-restricted binding of scFvCD7:Galectin-1 was obtained using T-leukemic Jurkat cells. Incubation of Jurkat cells with scFvCD7:Galectin-1 resulted in a strong binding (solid line) of the conjugate compared to antibody control (solid fill). Pre-incubation with CD7-blocking MAb TH-69 strongly inhibited binding of scFvCD7:Galectin-1 (dotted line). **B:** Binding of scFvC54:Galectin-1 was investigated using Jurkat cells expressing human EGP2 (Jurkat.EGP2). Conjugate scFvC54:Galectin-1 contains the EGP2-selective antibody fragment scFvC54. EGP2 is an established target antigen, previously used in antibody-based therapies, that is selectively expressed on the cell surface of various human carcinomas, but not on wild-type Jurkat cells. Incubation of Jurkat.EGP2 with scFvC54:Galectin-1 resulted in strong and selective binding to the cell surface (solid line), whereas no binding was detected on parental EGP2-negative Jurkat cells (solid fill).
**Fig 6. Target antigen-restricted apoptosis induction by tumor-retargeted scFv:Galectin-1 conjugates. A:** Treatment of Jurkat cells for 16h with anti-CD7 scFv:Galectin-1 (<1 µM) resulted in a potent induction of apoptosis in up to approximately 80% of the cells. Treatment with high concentrations of conventional recombinant Galectin-1 (rGal-1; 20 µM) only minimally induced apoptosis. **B:** Jurkat cells, retrovirally transduced with the pan-carcinoma marker EGP2 (white bars) were treated with the EGP2 targeting conjugate scFvC54:Galectin-1 (<1 µM), which resulted in a potent induction of apoptosis. Parental Jurkat cells (black bars) were not sensitive to treatment with anti-EGP2 scFv:Galectin-1.

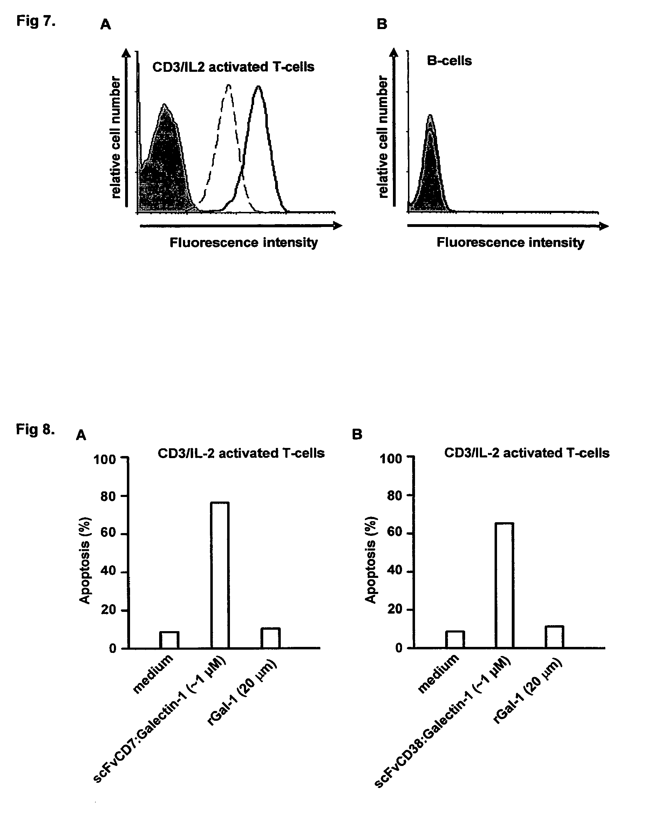

**Fig 7. Target antigen restricted binding of T-cell specific seFv:Galectin-1. A:** Incubation of anti-CD3/IL-2 activated T-cells with anti-CD7 scFv:Galectin-1 results in strong binding to the cell surface (solid line), compared to antibody control (solid fill). Binding is competitively inhibited by co-incubation of with target antigen-blocking MAb (dotted line). **B:** No binding is detected on target antigen-negative cells. Incubation with scFv:Galectin-1 (solid line) results in a fluorescent intensity comparable to antibody control (solid fill).
**Fig. 8. Target antigen restricted induction of apoptosis on activated T-cells by T-cell specific scFv:Galectin-1.** Activated T-cells were treated with **A:** anti-CD7 or **B:** anti-CD38 scFv:Galectin-1, after which induction of apoptosis was assessed by Annexin V/ propidium iodide (PI) staining using flow cytometry. Treatment with anti-CD7 or anti-CD38 scFv:Galectin-1 (appr. 1 µM) resulted in a strong increase in apoptosis in activated T-cells compared to medium. Treatment with conventional recombinant Galectin-1 (20 µM) did not induce apoptosis.

### EXPERIMENTAL SECTION

To demonstrate the enhanced biological efficacy of a conjugated galectin molecule, three different scFv:Galectin-1 conjugates were generated and
characterized in more detail with respect to their capacity to induce apoptosis. The target cell-surface antigens involved in present study were: human CD7, human CD38, and human EGP2. CD7 and CD38 are both established leukemia-associated target antigens and antigens highly expressed on activated T-cells. Epithelial Glycoprotein-2 (EGP2) is an established pan-carcinoma target antigen.

### Materials and Methods

### Monoclonal antibodies and scfv antibody fragments

MAb NCL-Gal1, a murine IgG1 with specificity for human galectin-1, was purchased from Novocastra Laboratories, UK. MAb TH69 is a murine IgG1 with specificity for human CD7 and was kindly provided by Prof. Dr. Martin Gramatzki, Division of Stem Cell and Immunotherapy, 2nd Medical Department, University Clinic Schleswig-Holstein, Kiel, Germany.

MAb MOC31 is a murine IgG1 with high affinity for human EGP2.

The scFvCD38 was generated using available sequence data.

Phagemid pCANTAB5E/scFv3A1F encoding anti-CD7 antibody fragment 3A1F was kindly provided by Dr. Chris Pennell, Department of Laboratory Medicine and Pathology, University of Minnesota. MAb TH69 and scFv-3A1F compete for binding to the same or overlapping epitope on the extracellular domain of human CD7.

Phagemid pCANTAB6E/scFvC54 encoding anti-EGP2 antibody fragment C54 was kindly provided by Prof. Logtenberg, University of Utrecht. MAb MOC31 and scFvC54 compete for binding to the same or overlapping epitope on the extracellular domain of human EGP2.

### Cell lines

The human CD7-positive T-ALL cell line Jurkat and the CD7-negative human B-cell lymphoma cell lines Ramos were purchased from the ATCC (Manassas, USA). T-cell line MOLT16 was a kind gift of Prof. Dr. Martin Gramatzki. An EGP2-positive transfectant of the Jurkat cell line was generated as previously described (Bremer et al, IJC, 2004, 109; 281-290, 2004. All cell lines were cultured in RPMI (Cambrex, New Jersey, New Hampshire, USA) supplemented with 13% FCS, at 37°C in humidified 5% CO2 atmosphere.

### Activated T-cells

Peripheral blood lymphocytes (PBLs) were isolated from whole blood of healthy donors by standard density gradient centrifugation procedures (Lymphoprep, Axis-Shield PoC As., Oslo, Norway). Freshly isolated PBLs were resuspended at 2.0 x 10*6 cell/ml in RPMI, supplemented with 10% human pooled serum. Activated T-cells were obtained by incubation of freshly isolated PBLs with anti-CD3 MAb WT32 (0.5 µg/ml) for 72 h, followed by IL-2 stimulation (100 ng/ml) for 48 h.

### Construction of scFv:Galectin-1 conjugates

Full-length human galectin-1 cDNA was kindly provided by Dr. Linda Baum, Department of Pathology and Laboratory Medicine, UCLA School of Medicine, Los Angeles, California 90095, USA. Previously, we described the eukaryotic expression plasmid pEE14scFv:sTRAIL for the rapid construction, evaluation and stable expression of scFv:sTRAIL fusion proteins in CHO-K1 cells (Bremer et al, Int. J. of Cancer, 109; 281-290, 2004). Important features of this vector are the presence of the murine kappa light-chain leader peptide encoded upstream of 2 multiple cloning sites (MCSs) that are separated by a 26 residue in-frame linker sequence, and the glutamine synthetase selectable marker gene, which allows for amplified expression of the recombinant protein in the established production cell line CHO-K1. The vector exploits the strong CMV promoter to drive recombinant protein expression, while the leader peptide directs excretion of the fusion protein into the culture supernatant. In the first MCS, DNA fragments encoding either anti-CD7, anti-EGP2 , or anti-CD38 scFv's were directionally inserted using the unique *Sfi*I and *Not*I restriction enzyme sites. In the second MCS, the sTRAIL encoding cDNA was replaced for a PCR-modified 438 bp DNA fragment encoding human Galectin-1 using restriction enzymes *Xho*I and *Xba*I and standard DNA manipulation procedures. This resulted in the plasmid pEE14-scFv:Galectin (Fig.2). Galectin-1 cDNA manipulation was performed by PCR using proofread DNA polymerase according to standard protocol using primers:
T1: 5'- ATCCTCGAGTCTAGTGGGAGCGGATCTGCTTGTGGTCTGGTCGCC - 3' (*Xho*I site is underlined) and T2: 5' TCTAGATCAGTCAAAGGCCACACATT TGATCTT-3' (*Xba*I site is underlined).

Plasmid pEE14 scFv:Galectin can be used for the rapid and combinatorial construction of a series of different scFv:Galectin conjugates. This series includes conjugates in which the tandem order of scFv and Galectin is reversed, yielding conjugates in which a given galectin is inserted in MCS #1 followed by the linker sequence and a given scFv inserted in MCS #2. Moreover, the plasmid platform allows for insertion of a (homo or hetero)-dimerization domain either N-terminally or C-terminally to the fusion protein or, if necessary, in the linker region. The HA-tag is derived from the human influenza hemagglutinin (HA) protein and commercially available anti-HA immune-affinity matrix can be used to purify HA-tagged scFv:Galectin conjugates under native conditions from conditioned cell culture media. Alternatively, purification of galectin conjugates can be performed under native conditions using α-galactosyl agarose resins.

### Production of scFv:Galectin-1

The various scFv:Galectin-1 fusion proteins were expressed in CHO-K1 cells with the glutamine synthetase selection/amplification system. Briefly, CHO-K1 cells were transfected with the various pEE14scFv:galectin-1 using Fugene-6 reagent (Roche Diagnostics, Almere, The Netherlands). Stable transfectants with amplified expression were isolated and single cell sorted with a high-speed cell sorter (Cytomation, Fort Collins, USA). Individual clones were assessed for stable and high secretion of scFv:Galectin-1 in the absence of the MSX selection reagent. ScFv:Galectin-1 containing medium was harvested (10.000 g, 10 min).

### Target antigen-specific binding of scFv:Galectin-1

EGP2-specific binding of scFvC54:Galectin-1 was assessed by incubation of 1.0x10⁶ Jurkat.EGP2 cells with scFvC54:Galectin-1 containing medium in the presence or absence of EGP2-blocking MAb MOC31 (5 µg/ml). EGP2-specific binding was analyzed by flow cytometry. Incubations were carried out for 45 min at 0°C and were followed by two washes with serum free medium. CD7 or CD38 restricted binding of scFvCD7:Galectin-1 and scFvCD38:Galectin-1 was assessed using similar procedures using MOLT16 (CD7-positive) and Jurkat cells (CD7 and CD38-positive).

### Target antigen-restricted apoptosis induction by scFv:Galectin-1

Tumor cells were seeded at 0.5x10⁶ cells/well in a 48-well plate and treated for 16 h with the indicated concentrations of the respective scFv:Galectin-1 in the presence or absence of target antigen blocking MAb (5 µg/ml).

Apoptosis was assessed by measuring cell surface appearance of phosphatidyl serine (PS) or by loss of mitochondrial membrane potential (ΔΨ). PS exposure was determined using flow cytometric analysis with an AnnexinV-FITC/PI kit (VPS Diagnostics, Hoeven, The Netherlands) according to manufacturer's instructions. ΔΨ was analyzed with the cell-permeant green-fluorescent lipophilic dye DiOC6 (Molecular Probes, Eugene, USA). After treatment, cells were harvested by centrifugation (300g, 5 min), incubated for 20 min at 37 °C with 0.1 µM DiOC6 in fresh medium, washed once with PBS, and analyzed by flow cytometry.

### Results

### Tumor-selective induction of apoptosis by scFv:Galectin-1 conjugates

Specific binding of scFvCD7:Galectin-1 was analyzed using the acute T-cell leukemia cell line Jurkat, which highly expresses the antigen CD7. Jurkat cells were incubated with supernatant containing the galectin-conjugate scFvCD7:Galectin-1. Subsequently, binding of the conjugate was analyzed by flow cytometry using the anti-Galectin-1 antibody NC1-Gal and appropriate secondary PE-conjugated antibody (Fig. 5A). Incubation of Jurkat cells with scFvCD7:Galectin-1 clearly resulted in a strong increase in fluorescent intensity (solid line) compared to antibody control alone (solid fill). Pre-incubation with CD7-blocking MAb TH-69 inhibited binding of scFvCD7:Galectin-1. Furthermore, no binding was detected on CD7-negative Ramos cells (data not shown). Together, this indicates that scFvCD7:Galectin-1 strongly binds to CD7 on the cell surface of target cells only. Since galectin-1 mediated binding to the cell surface on CD7-negative Ramos cells was below detection levels, this further indicates that the conjugate predominantly binds via the CD7-specific cell targeting means.

Similar results were obtained with the EGP2-specific scFvC54:Galectin-1 conjugate (Fig. 5B) using EGP-2-transduced Jurkat cells (Jurkat-EGP2). Incubation of Jurkat-EGP2 resulted in a strong binding of the conjugate to the cell surface (solid line) compared to incubation of parental Jurkat cells not expressing EGP-2 with scFvC54:Galectin-1 (solid fill).

Next, the apoptotic activity of the conjugates scFvCD7:Galectin-1 and scFvC54:Galectin-1 was analyzed. The acute T-cell leukemic cell line Jurkat, strongly positive for CD7, was treated with scFvCD7:Galectin-1 (appr. 1 µM) or conventional recombinant Galectin-1 (20 µM) for 24 hours (Fig. 6A). This procedure revealed a potent induction of apoptosis in scFvCD7:Gal-1 treated cells, compared to medium control levels. Importantly, conventional recombinant Galectin-1 minimally induced apoptosis at approximately 10-fold higher concentrations, indicating the superior apoptotic activity of the scFvCD7:Galectin-1 conjugate compared to non-targeted Galectin-1.

In a similar experiment, the apoptotic activity of scFvC54:Galectin-1 was investigated using EGP2 positive Jurkat.EGP2 cells and parental EGP2 negative Jurkat cells (Fig. 6B). Treatment of Jurkat.EGP2 for 24 hours with scFvC54:Galectin-1 resulted in a strong increase in apoptosis compared to medium control, whereas treatment of parental EGP2 negative Jurkat cells did not result in a significant increase in apoptosis.

### Anti-inflammatory activity of novel T-cell retargeted scFv:Galectin-1 conjugates.

To investigate the potential of scFv:Galectin-1 for the resolution of T-cell mediated autoimmunity, a scFv:Galectin-1 conjugates with specificity for the antigen CD7 and CD38 were generated as described above. CD7 and CD38 are highly expressed, specifically on activated T-cells. Binding of scFvCD7:Galectin-1 was assessed on activated T-cells using flow cytometry. Incubation of activated T-cells with scFvCD7:Galectin-1 clearly resulted in a strong binding to the cell surface (Fig. 7A; solid line) compared to antibody control (Fig. 7A; solid fill). Pre-incubation with CD7-blocking MAb TH-69, strongly inhibited binding of scFvCD7:Galectin-1 (Fig. 7A; dotted line), indicating that binding is CD7-mediated. No binding was detected on CD7-negative B-cells (Fig. 7B; antibody control, solid fill; scFvCD7:Galectin-1, solid line), indicating the enhanced specificity of scFvCD7:Galectin-1 for activated T-cells. Similar results were obtained for the scFvCD38:Galectin-1 conjugate (data not shown).

Subsequently, the apoptotic activity of scFvCD7:Galectin-1 towards activated T-cells was analyzed. Treatment of activated T-cells with scFvCD7:Galectin-1 (appr. 1µM) for 24h resulted in a potent induction of apoptosis up to approximately 80% (Fig. 8A). Importantly, only minimal apoptotic activity was found when activated T-cells were treated with conventional, non-conjugated recombinant Galectin-1 at a very high concentration (20 µM), indicating the enhanced apoptotic activity of scFvCD7:Galectin-1 towards activated T-cells. Similarly, scFvCD38:Galectin-1 also potently induced apoptosis in activated T-cells (Fig. 8B), whereas conventional recombinant galectin-1 did not induce apoptosis.

## Claims

1. A galectin-conjugate comprising at least one galectin molecule conjugated to a non-galectin cell targeting means.

2. Galectin-conjugate according to claim 1, wherein said targeting means is a proteinaceous substance.

3. Galectin-conjugate according to claim 1 or 2, wherein said galectin molecule is genetically fused to said targeting means, optionally via a linker sequence.

4. Galectin-conjugate according to claim 1 or 2, wherein said galectin molecule is chemically coupled to said targeting means.

5. Galectin-conjugate according to any one of claims 1 to 4, wherein said targeting means allows for cancer-cell specific targeting.

6. Galectin-conjugate according to any one of the above claims, wherein said targeting means is capable of binding to a target cell surface molecule selected from the group consisting of cluster of differentiation (CD) antigens, ABC transporter proteins, ligands that are overexpressed on tumor cells, adhesion molecules, ligands that are expressed exclusively or predominantly on one or more types of leucocytes, one or more sub-populations of T cells or B cells, cytokine receptors, growth factor receptors, molecules or complex comprising a transmembrane protein, tyrosine kinase type receptors, serine kinase type receptors, heterotrimetric G-protein coupled receptors, receptors bound to tyrosine kinase, TNF family receptors, notch family receptors, guanylate cyclase types, tyrosine phosphatase types, decoy receptors, inhibitory receptors and adhesion receptors or any cell surface receptor-ligand cooperating with such a receptor, preferably wherein said targeting means is capable of binding to a CD antigen, such as CD7 or CD38, or a TNF family member, such as TRAIL-R.

7. Galectin-conjugate according to any one of claims 1 to 6, wherein said targeting means comprises an antibody or a functional fragment thereof, preferably a single chain variable antibody fragment (scFv).

8. Galectin-conjugate according to any one of claims 1 to 7, wherein said at least one galectin molecule is conjugated to a bi- or multi-functional targeting means.

9. Galectin-conjugate according to any one of claims 1 to 8, wherein said at least one galectin molecule is biologically active as a dimer.

10. Galectin-conjugate according to any one of claims 1 to 9, wherein said galectin molecule is a mutated galectin molecule.

11. Galectin-conjugate according to any one of claims 1 to 10, wherein said galectin-conjugate comprises a dimer of galectin molecules, at least one of which is conjugated to a cell targeting means, preferably wherein the galectin molecules of said dimer are covalently linked, such as via a GG or SS- linker, or stabilized via dimerization domains, such as leucine zipper domains.

12. Galectin-conjugate according to claim 11, wherein the two galectin molecules are each conjugated to a cell targeting means.

13. Galectin-conjugate according to claim 12, wherein said galectin molecules are conjugated to identical or to distinct cell targeting means.

14. A pharmaceutical composition comprising a galectin-conjugate according to any one of claims 1-13 and a pharmaceutically acceptable carrier.

15. The use of a galectin-conjugate according to any one of claims 1-13 for the manufacture of a medicament for the treatment of an inflammatory disease or for the treatment of cancer.

16. The use of a galectin-conjugate according to any one of claims 1-13 as an apoptosis-inducing or immunosuppressive agent.

17. A method for the treatment or profylaxis of a condition that would benefit from galectin therapy, comprising administering to a subject in need thereof a therapeutically effective dose of a pharmaceutical composition according to claim 14, preferably wherein said condition is an immune disorder, more preferably an immune disorder selected from the group consisting of an acute or chronic inflammatory disease, such as auto-immune disease, allergic disorder, autoimmune encephalomyelitis, arthritis, colitis, hepatitis, asthma, multiple sclerosis, transplant rejection, Graft-versus-host disease (GVHD) and inflammatory bowel disease.

18. A method for the treatment of cancer, comprising administering to a subject in need thereof a therapeutically effective dose of a pharmaceutical composition according to claim 14.
